# EUROPEAN PATENT SPECIFICATION

(11) **EP 4 352 066 B1**
(45) Date of publication and mention of the grant of the patent: **06.08.2025**
(21) Application number: 22757197.3
(22) Date of filing: 10.06.2022
(51) Int. Cl.: C07F 1/00, C07F 13/00, C07F 15/02, C07F 15/04, C07F 15/06, C07F 3/00, A01N 63/20, A61P 21/00

(54) **CHELATED NUTRIENTS AND METHODS OF USE THEREOF**
CHELATIERTE NÄHRSTOFFE UND VERFAHREN ZUR VERWENDUNG DAVON
NUTRIMENTS CHÉLATÉS ET LEURS PROCÉDÉS D'UTILISATION

(30) Priority: 10.06.2021 US 202163202410 P
(43) Date of publication of application: 17.04.2024
(73) Proprietor: ICL AMERICA DO SUL S.A., Pinheiros, 05425-070, São Paulo, SP (BR)
(72) Inventor: ORUI SAITO, Bruno, 13140-580 Paulínia - SP (BR); CLEMENTE VITTI, Daniela Cristina, 13405-090 Piracicaba - SP (BR); CASTELLANI DA ROCHA, Michel, 13414-424 Piracicaba - SP (BR); MORAES CASTANHO DE ALMEIDA, Maria Emilia, 08840-550 Mogi das Cruzes - SP (BR); MARQUES DA SILVA, Keila Cristina, 08660-048 Suzano - SP (BR)
(74) Representative: Reddie & Grose LLP
(86) International application number: PCT/BR2022/050210
(87) International publication number: WO 2022/256897

(56) References cited:
- EP-A2- 0 308 983
- WO-A1-2020/247824
- US-A- 4 322 361

## Description

### FIELD OF THE DISCLOSURE

The field of the disclosure relates generally to chelated nutrients and methods and use thereof. More specifically, the disclosure is directed to formulations comprising new nutrients chelated for agronomical applications.

### BACKGROUND OF THE DISCLOSURE

The most common chelating agents (i.e., precursors of chelates) today are EDTA (ethylenediamine tetraacetic acid) and EDDHA (ethylenediaminedi (o-hydroxyphenylacetic) acid). While market demand is high for innovative products that provide a guaranteed source of nutrients for agronomical application, EDTA is most commonly applied for this purpose and already dominates manufacturing technology in several countries. However, there is no record of product biodegradability and bioavailability of conventional chelates within the plant when absorbed.

Accordingly, there is a need for biodegradable and bioavailable chelating agents capable of providing nutrients concentrations in agronomical applications that are also efficient, compatible with other crops products and cost effective, and for instance can be used in addition to or as an alternative to conventional chelates and chelating agents.

WO 2020/247824 A1 discloses agricultural formulations comprising living microorganisms and an elevated concentration of a chelating agent. Formulation 1. of the Examples comprises 75% of Fe-HEDTA solution, 0.5% *B. amyloliquefaciens,* 4.50% urea, 19% seaweed-derived biostimulant, 1% water. Formulation 2. of the Examples comprises 75% of Zn-EDTA solution, 0.5% *B. amyloliquefaciens,* 4.50% urea, and 20% leonardite-derived biostimulant.

### BRIEF DESCRIPTION OF THE DISCLOSURE

In one aspect, the present disclosure is directed to a chelate A, having a structure [M(Ligand 1)(Ligand 2)]2Y, wherein: M is a metal selected from the group consisting of Cu, Mn, Zn, Co, Ni, Mg, Ca and Fe; Ligand 1 is glutamic acid; Ligand 2 is malic acid; and Y is a base cation, wherein the base is preferably selected from the group consisting of NaOH,KOH and NH4OH; and wherein a binder proportion ratio of glutamic acid to malic acid to metal is 1:1:1.

In another aspect, the present disclosure is directed to a chelate B, having a structure [M(Ligand 1)(Ligand 2)]2Y, wherein: M is a metal selected from the group consisting of Cu, Mn, Zn, Co, Ni, Mg, Ca and Fe; Ligand 1 is glutamic acid; Ligand 2 is aspartic acid; and Y is a base cation, wherein the base is preferably selected from the group consisting of NaOH,KOH and NH4OH; and wherein a binder proportion ratio of glutamic acid to aspartic acid to metal is 1:1:1.

In some embodiments of the chelate, Cu is present in an amount between 5 to 7% (liquid form), Mn is present in an amount between 5 to 7% (liquid form), Zn is present in an amount between 5 to 7% (liquid form), Mg is present in an amount between 1.5 to 3.5% (liquid form), Co is present in an amount between 3.5 to 5.5% (liquid form), Ni is present in an amount between 3.5 to 5.5% (liquid form), Ca is present in an amount between 5 to 7% (liquid form), and/or Fe is present in an amount between 5 to 7% (liquid form). In some embodiments, Cu is present in an amount between 13 to 16.5% (solid form), Mn is present in an amount between 13 to 16.5% (solid form), Zn is present in an amount between 13 to 16.5% (solid form), Mg is present in an amount between 6 to 7% (solid form), Co is present in an amount between 13 to 16.5% (solid form), Ni is present in an amount between 13 to 16.5% (solid form), Ca is present in an amount between 13 to 16.5% (solid form), and/or Fe is present in an amount between 13 to 16.5% (solid form).

In another aspect, the present disclosure is directed to a formulation for agronomical application , the formulation comprising: a chelate A having a structure [M(Ligand 1)(Ligand 2)]2Y, wherein: M is a metal selected from the group consisting of Cu, Mn, Zn, Co, Ni, Mg, Ca and Fe; Ligand 1 is glutamic acid; Ligand 2 is malic acid; and Y is a base cation, wherein the base is preferably selected from the group consisting of NaOH, KOH and NH4OH; and water; wherein a binder proportion ratio of glutamic acid to malic acid to metal is 1:1:1.

In another aspect, the present disclosure is directed to a formulation for agronomical application , the formulation comprising: a chelate B having a structure [M(Ligand 1)(Ligand 2)]2Y, wherein: M is a metal selected from the group consisting of Cu, Mn, Zn, Co, Ni, Mg, Ca and Fe; Ligand 1 is glutamic acid; Ligand 2 is aspartic acid; and Y is a base cation, wherein the base is preferably selected from the group consisting of NaOH, KOH and NH4OH; and water; wherein a binder proportion ratio of glutamic acid to aspartic acid to metal is 1:1:1.

In another aspect, the present disclosure is directed to a formulation for agronomical application , the formulation comprising a combination of the chelates A and B.

In some embodiments of the formulation, Cu is present in an amount between 5 to 7% (liquid form), Mn is present in an amount between 5 to 7% (liquid form), Zn is present in an amount between 5 to 7% (liquid form), Mg is present in an amount between 1.5 to 3.5% (liquid form), Co is present in an amount between 3.5 to 5.5% (liquid form), Ni is present in an amount between 3.5 to 5.5% (liquid form), Ca is present in an amount between 5 to 7% (liquid form), and/or Fe is present in an amount between 5 to 7% (liquid form). In some embodiments, Cu is present in an amount between 13 to 16.5% (solid form), Mn is present in an amount between 13 to 16.5% (solid form), Zn is present in an amount between 13 to 16.5% (solid form), Mg is present in an amount between 6 to 7% (solid form), Co is present in an amount between 13 to 16.5% (solid form), Ni is present in an amount between 13 to 16.5% (solid form), Ca is present in an amount between 13 to 16.5% (solid form), and/or Fe is present in an amount between 13 to 16.5% (solid form).

In yet another aspect, the present disclosure is directed to a method of applying an agronomical formulation, the method comprising: preparing a formulation comprising a water soluble chelate A and/or B having a structure [M(Ligand 1)(Ligand 2)]2Y, wherein: M is a metal selected from the group consisting of Cu, Mn, Zn, Co, Ni, Mg, Ca and Fe. The method further comprises applying the formulation via at least one of a foliar application, a seed application, and a soil application.

In some embodiments of the method, Cu is present in an amount between 5 to 7% (liquid form), Mn is present in an amount between 5 to 7% (liquid form), Zn is present in an amount between 5 to 7% (liquid form), Mg is present in an amount between 1.5 to 3.5% (liquid form), Co is present in an amount between 3.5 to 5.5% (liquid form), Ni is present in an amount between 3.5 to 5.5% (liquid form), Ca is present in an amount between 5 to 7% (liquid form), and/or Fe is present in an amount between 5 to 7% (liquid form). In some embodiments, Cu is present in an amount between 13 to 16.5% (solid form), Mn is present in an amount between 13 to 16.5% (solid form), Zn is present in an amount between 13 to 16.5% (solid form), Mg is present in an amount between 6 to 7% (solid form), Co is present in an amount between 13 to 16.5% (solid form), Ni is present in an amount between 13 to 16.5% (solid form), Ca is present in an amount between 13 to 16.5% (solid form), and/or Fe is present in an amount between 13 to 16.5% (solid form).

### BRIEF DESCRIPTION OF THE DRAWINGS

The embodiments described herein may be better understood by referring to the following description in conjunction with the accompanying drawings.
FIG. 1 is an exemplary embodiment of a reaction scheme in accordance with the present disclosure, chelate A.
FIG. 2 is an exemplary embodiment of obtained chelates in solution in accordance with the present disclosure.
FIG. 3 is an exemplary embodiment of a compatibility test in accordance with the present disclosure.
FIG. 4 is an exemplary embodiment of a corrosion test in accordance with the present disclosure.
FIG. 5 is an exemplary embodiment of Mn content of old leaves in accordance with the present disclosure.
FIG. 6 is an exemplary embodiment of Mn content of new leaves in accordance with the present disclosure.
FIG. 7 is an exemplary embodiment of Mn translocation comparing EDTA chelates and Glu+ Mal chelates in accordance with the present disclosure.
FIG. 8 is an exemplary embodiment of micronutrient guarantees of water-soluble formulations comprising a zinc chelate in accordance with the present disclosure.
FIG. 9 is an exemplary embodiment of a reaction scheme in accordance with the present disclosure, chelate B.

### DETAILED DESCRIPTION OF THE DISCLOSURE

In exemplary embodiments, the present disclosure describes a foliar nutrient product that incorporates two organic ligands forming a chelate with nutrients (e.g., copper (Cu), manganese (Mn), zinc (Zn), nickel (Ni), cobalt (Co), magnesium (Mg), calcium (Ca) and Iron (Fe)), and forming fully water-soluble metal chelates to be applied as suitable chelated nutrients in water-soluble formulations of interest. Particularly, embodiments of the present disclosure describe glutamate-malate and/or glutamate-aspartate chelates that are in general smaller than EDTA chelates and that are more concentrated with nutrients than EDTA chelates. More concentrated products are a market requirement to reduce the cost of transportation and storage. Besides that, glutamate-malate and/ or glutamate-aspartate chelates have equivalent characteristics to EDTA chelates with respect to stability, pH, bonding strength, solubility, and compatibility, e.g., for use as a satisfactory EDTA chelates alternative for providing nutrients in agronomical applications.

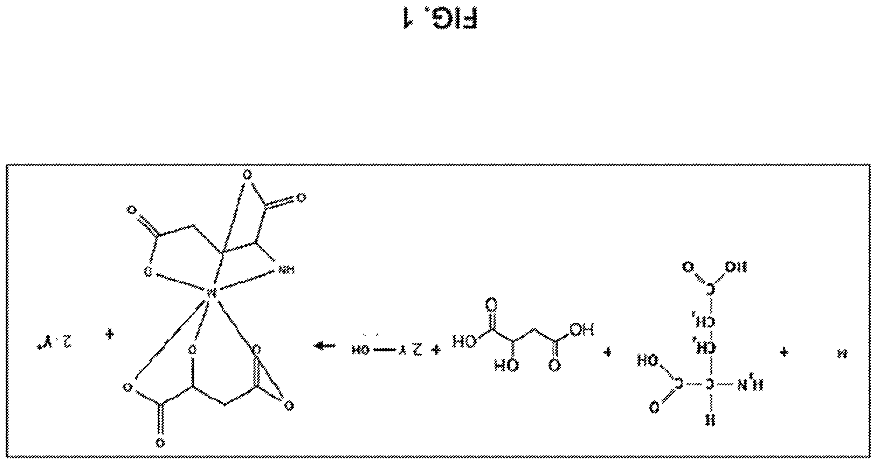

Turning now to FIG. 1, the illustrated reaction scheme depicts the general chelation reaction described herein. In exemplary embodiments, the chelates have the following structure: [M(Ligand 1)(Ligand 2)] 2Y, where:
M = metal (Cu, Mn, Zn, Co, Ni, Mg, Ca and Fe) without a counter ion (for a chelation reaction, the metal is provided as a metal carbonate (CO₃²⁻) or metal oxide (O²⁻) reagent as described herein below);
Ligand 1 = glutamic acid;
Ligand 2 = malic acid; and
Y = base proven cation used for final solution pH adjustment (e.g., NaOH,KOH or NH4OH).

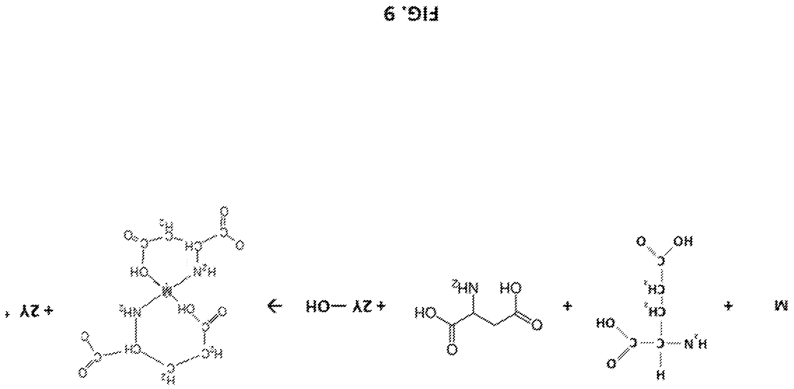

Turning now to FIG. 9, the illustrated reaction scheme depicts the general chelation reaction described herein. In exemplary embodiments, the chelates have the following structure: [M(Ligand 1)(Ligand 2)] 2Y, where:
M = metal (Cu, Mn, Zn, Co, Ni, Mg, Ca and Fe) without a counter ion (for a chelation reaction, the metal is provided as a metal carbonate (CO₃²⁻) or metal oxide (O²⁻) reagent as described herein below);
Ligand 1 = glutamic acid;
Ligand 2 = aspartic acid; and
Y = base proven cation used for final solution pH adjustment (e.g., NaOH, KOH or NH4OH).

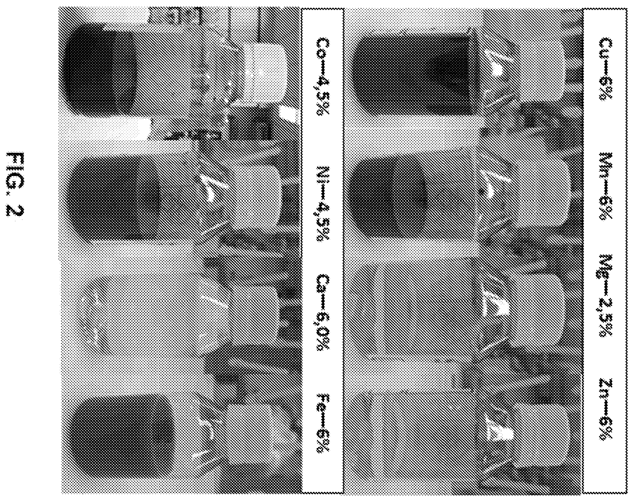

The chelation reactions were carried out with carbonate salts of Cu, Mn, Co, Ni, Mg and Ca metals and oxide for Zn and Fe metals. All salts became soluble when complexed with glutamic and malic acid as 1: 1: 1, metal: binder proportion (see FIG. 2), or glutamic and aspartic acid as 1: 1: 1, metal: binder proportion That is, a binder proportion ratio of glutamic acid to malic acid to metal is 1:1:1. FIG. 2 illustrates the obtained chelates of Cu at 6%, Mn at 6%, Mg at 2.5%, Zn at 6%, Ca at 6%, Fe at 6%, Co at 4.5%, and Ni at 4.5% in solution and/or with glutamic and aspartic acid After chelation was completed, each developed chelate (e.g., when applied as liquid or dried), was suitable for use as an isolated source of nutrient(s) or combined into water-soluble formulations that enhance the nutritional action with the insertion of other raw materials (e.g., such as the use of the Zn chelate in the formulation of Tonus^{®} or Kellus Blindex^{®}, registered commercial products). In exemplary embodiments, the chelates described herein are suitable for agronomical applications including foliar applications and/or drip irrigation applications.

For liquid forms, the Cu, Mn, Zn, Ca and/or Fe can be present in an amount between 5 to 7% including 5.1, 5.2, 5.3, 5.4, 5.5, 5.6, 5.7, 5.8, 5.9. 6, 6.1, 6.2, 6.3, 6.4, 6.5, 6.6, 6.7, 6.8, 6.9, and 7%. For liquid forms, the Mg can be present in an amount between 1.5 and 3.5% including 1.5, 1.6, 1.7, 1.8, 1.9, 2, 2.1, 2.2, 2.3, 2.4, 2.5, 2.6, 2.7, 2.8, 2.8, 3, 3.1, 3.2, 3.3, 3.4, and 3.5%. For liquid forms, the Co and or Ni can be present in an amount between 3.5 and 5.5% including 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, and 5.5%. Additionally, for liquid forms, the percentage(s) provided are calculated as mass solute/mass solution.

For solid forms (dried solution), the Cu, Mn, Zn, Ca, Co, Ni and/or Fe can be present in an amount between 13 to 16.5% including 13.1, 13.2, 13.3, 13.4, 13.5, 13.6, 13.7, 13.8, 13.9, 14, 14.1, 14.2, 14.3, 14.4, 14.5, 14.6, 14.7, 14.8, 14.9, 15, 15.1, 15.2, 15.3, 15.4, 15.5, 15.6, 15.7, 15.8, 15.9, 16, 16.1, 16.2, 16.3, 16.4 and16.5. For solid forms (spray dried solution), the Mg can be present in an amount between 6.0 and 7.0% including 6.1, 6.2, 6.2, 6.3, 6.4, 6.5, 6.6, 6.7, 6.8, 6.9 and 7.0%.

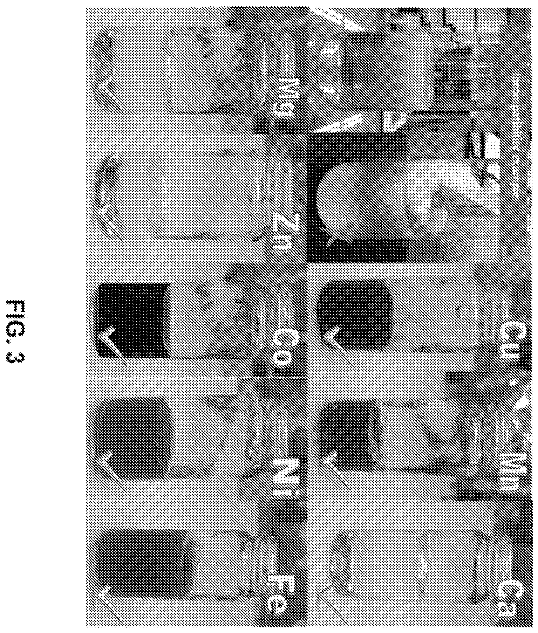

As shown in FIG. 3, compatibility tests were performed with the pesticide Agricultural Glyphosate with satisfactory physical results. Each of the Cu, Mn, Mg, Zn, Co, Ca, Fe and Ni chelates remained in solution.

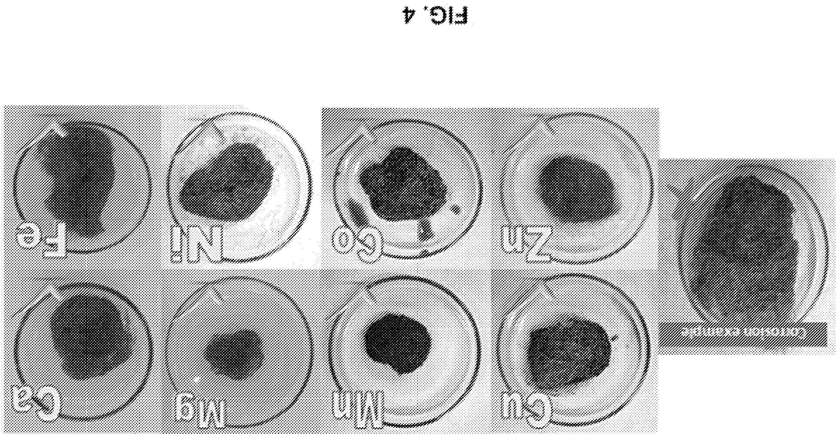

As shown in FIG. 4, corrosion tests were performed for each chelate. Assuming that a neutral product of any metal does not consume steel wool in the presence of the solution, corrosion testing on the steel wool was performed to verify that the entire structure of the chelate was completed with no free charges. (see FIG. 4).

Stability testing at different pH levels was performed and turning to Table 1, there is a large range of the chelates' stability, wherein the structure of the chelates can be considered stable.

**Table 1. Stability pH of the chelates**

| Metals | Chelate A | | Chelate B | |
|---|---|---|---|---|
| | Acid medium | Alkaline medium | Acid medium | Alkaline medium |
| Cu²⁺ | 0.51 | 10.86 | 0.30 | 10.7 |
| Mn²⁺ | 0.41 | 9.85 | 0.38 | 9.9 |
| Mg²⁺ | 0.41 | 9.85 | 0.39 | 9.75 |
| Zn²⁺ | 0.20 | 11.30 | 0.18 | 10.80 |
| Co²⁺ | 1.35 | 10.95 | 0,85 | 10.45 |
| Ni²⁺ | 0.20 | 10.56 | 0.18 | 10.1 |
| Ca²⁺ | 0.68 | 10.64 | 0.55 | 10.21 |
| Fe²⁺ | 1.12 | 8.59 | 1,53 | 7,95 |

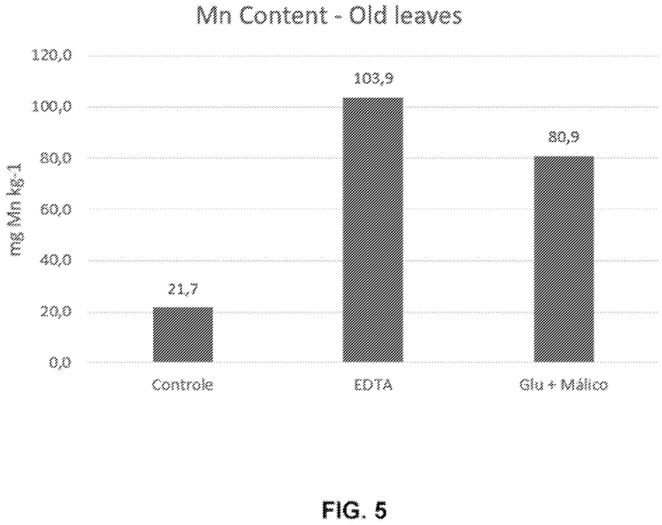

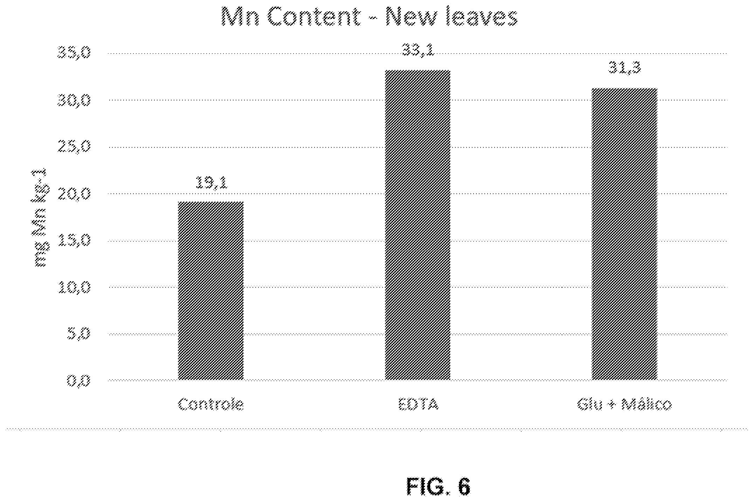

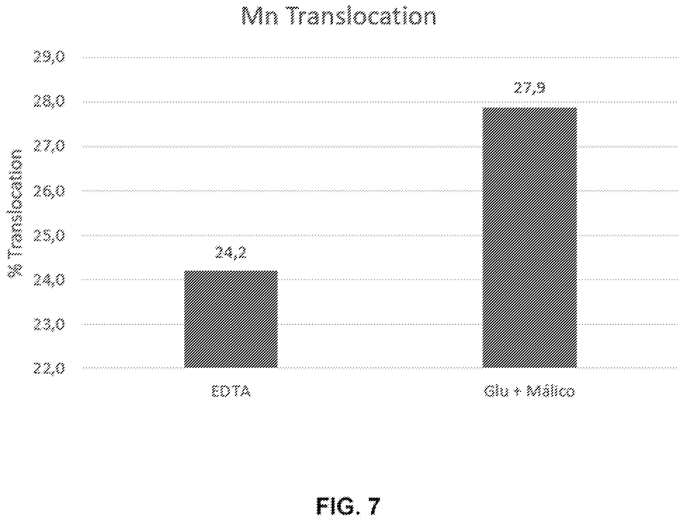

FIGs. 5 and 6 illustrate Mn content of old leaves and Mn content of new leaves, respectively. FIG. 7 shows Mn higher % of translocation which demonstrates nutrition and also metabolic recognition by dealing with organic chelating agents easily recognized by the plant.

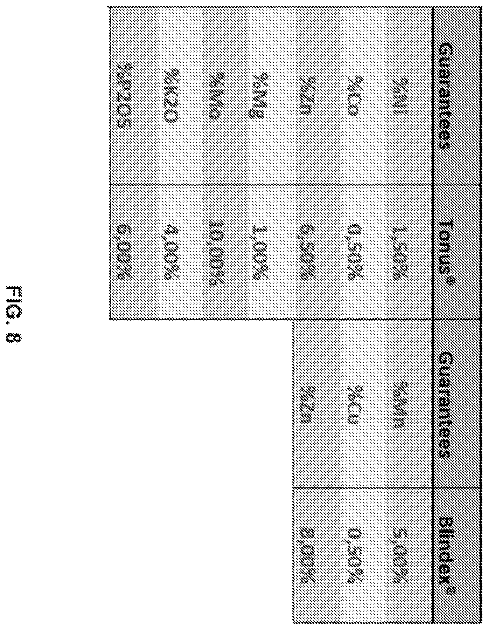

In some embodiments, the application of the chelates disclosed herein are further suitable in developing water-soluble formulations. Turning now to FIG. 8, the chart shows micronutrient guarantees of water-soluble formulations comprising a Zn chelate as described herein. For example, water-soluble formulations such as Tonus^{®} or Blindex^{®}, a mixture of salts including [Zn(Glu)(Mal)]2Na chelate as source of zinc and other micronutrient sources compound the guarantees exemplified in FIG. 8.

Definitions and methods described herein are provided to better define the present disclosure and to guide those of ordinary skill in the art in the practice of the present disclosure. Unless otherwise noted, terms are to be understood according to conventional usage by those of ordinary skill in the relevant art.

In some embodiments, numbers expressing quantities of ingredients, properties such as molecular weight, reaction conditions, and so forth, used to describe and claim certain embodiments of the present disclosure are to be understood as being modified in some instances by the term "about." In some embodiments, the term "about" is used to indicate that a value includes the standard deviation of the mean for the device or method being employed to determine the value. In some embodiments, the numerical parameters set forth in the written description and attached claims are approximations that vary depending upon the desired properties sought to be obtained by a particular embodiment. In some embodiments, the numerical parameters are to be construed in light of the number of reported significant digits and by applying ordinary rounding techniques. Notwithstanding that the numerical ranges and parameters setting forth the broad scope of some embodiments of the present disclosure are approximations, the numerical values set forth in the specific examples are reported as precisely as practicable. The numerical values presented in some embodiments of the present disclosure may contain certain errors necessarily resulting from the standard deviation found in their respective testing measurements. The recitation of ranges of values herein is merely intended to serve as a shorthand method of referring individually to each separate value falling within the range. Unless otherwise indicated herein, each individual value is incorporated into the specification as if it were individually recited herein.

In some embodiments, the terms "a" and "an" and "the" and similar references used in the context of describing a particular embodiment (especially in the context of certain of the following claims) are construed to cover both the singular and the plural, unless specifically noted otherwise. In some embodiments, the term "or" as used herein, including the claims, is used to mean "and/or" unless explicitly indicated to refer to alternatives only or to refer to the alternatives that are mutually exclusive.

The terms "comprise," "have" and "include" are open-ended linking verbs. Any forms or tenses of one or more of these verbs, such as "comprises," "comprising," "has," "having," "includes" and "including," are also open-ended. For example, any method that "comprises," "has" or "includes" one or more steps is not limited to possessing only those one or more steps and may also cover other unlisted steps. Similarly, any composition or device that "comprises," "has" or "includes" one or more features is not limited to possessing only those one or more features and may cover other unlisted features.

To facilitate the understanding of the embodiments described herein, a number of terms are defined below. The terms defined herein have meanings as commonly understood by a person of ordinary skill in the areas relevant to the present disclosure. Terms such as "a," "an," and "the" are not intended to refer to only a singular entity, but rather include the general class of which a specific example may be used for illustration. The terminology herein is used to describe specific embodiments of the disclosure, but their usage does not delimit the disclosure, except as outlined in the claims.

## Claims

1. A chelate A having a structure [M(Ligand 1)(Ligand 2)]2Y, wherein:
M is a metal selected from the group consisting of Cu, Mn, Zn, Co, Ni, Mg, Ca and Fe;
Ligand 1 is glutamic acid;
Ligand 2 is malic acid; and
Y is a base cation, wherein the base is selected from the group consisting of NaOH KOH and/or NH4OH,
wherein a binder proportion ratio of glutamic acid to malic acid to metal is 1:1:1.

2. A formulation for agronomical application, the formulation comprising:
a chelate A having a structure [M(Ligand 1)(Ligand 2)]2Y, wherein:
M is a metal selected from the group consisting of Cu, Mn, Zn, Co, Ni, Mg, Ca and Fe;
Ligand 1 is glutamic acid;
Ligand 2 is malic acid; and
Y is a base cation, wherein the base is selected from the group consisting of NaOH KOH and/or NH4OH; and
water,
wherein a binder proportion ratio of glutamic acid to malic acid to metal is 1:1:1.

3. A method of applying an agronomical formulation, the method comprising:
preparing a formulation comprising a water soluble chelate A having a structure [M(Ligand 1)(Ligand 2)]2Y, wherein:
M is a metal selected from the group consisting of Cu, Mn, Zn, Co, Ni, Mg, Ca and Fe;
Ligand 1 is glutamic acid;
Ligand 2 is malic acid; and
Y is a base cation, wherein the base is selected from the group consisting of NaOH KOH and/or NH4OH;
wherein a binder proportion ratio of glutamic acid to malic acid to metal is 1:1:1; and
applying the formulation via at least one of a foliar application, a seed application, and a soil application.

4. A chelate B having a structure [M(Ligand 1)(Ligand 2)]2Y, wherein:
M is a metal selected from the group consisting of Cu, Mn, Zn, Co, Ni, Mg, Ca and Fe;
Ligand 1 is glutamic acid;
Ligand 2 is aspartic acid; and
Y is a base cation, wherein the base is selected from the group consisting of NaOH and/or KOH; and
wherein a binder proportion ratio of glutamic acid to malic acid to metal is 1:1:1.

5. The chelate of claim 1 or 4, wherein at least one of the Cu, Mn, Zn, Fe and Ca are present in an amount between 5 to 7%, Mg is present in an amount between 1.5 to 3.5%, Co and Ni are present in an amount between 3.5 to 5.5% (liquid form).

6. The chelate of claim 1 or 4, wherein at least one of the Cu, Mn, Zn, Co, Ni, Fe and Ca are present in an amount between 13 to 16,5%, Mg is present in an amount between 6.0 to 7.0% (solid form).

7. A formulation for agronomical application, the formulation comprising:
a chelate B having a structure [M(Ligand 1)(Ligand 2)]2Y, wherein:
M is a metal selected from the group consisting of Cu, Mn, Zn, Co, Ni, Mg, Ca and Fe;
Ligand 1 is glutamic acid;
Ligand 2 is aspartic acid; and
Y is a base cation, wherein the base is selected from the group consisting of NaOH KOH and/or NH4OH; and
water;
wherein a binder proportion ratio of glutamic acid to malic acid to metal is 1:1:1.

8. A method of applying an agronomical formulation, the method comprising:
preparing a formulation comprising a water soluble chelate B having a structure [M(Ligand 1)(Ligand 2)]2Y, wherein:
M is a metal selected from the group consisting of Cu, Mn, Zn, Co, Ni, Mg, Ca and Fe;
Ligand 1 is glutamic acid;
Ligand 2 is aspartic acid; and
Y is a base cation, wherein the base is selected from the group consisting of NaOH KOH and/or NH4OH;
wherein a binder proportion ratio of glutamic acid to malic acid to metal is 1:1:1; and
applying the formulation via at least one of a foliar application, a seed application, and a soil application.

9. A formulation for agronomical application, the formulation comprising a mixture of a chelate A as defined in claim 1 and a chelate B as defined in claim 4.

10. The formulation of claim 2, 7 or 9, wherein at least one of the Cu, Mn, Zn, Fe and Ca are present in an amount between 5 to 7%, Mg is present in an amount between 1.5 to 3.5%, Co and Ni are present in an amount between 3.5 to 5.5% (liquid form).

11. The formulation of claim 2, 7 or 9, wherein at least one of the Cu, Mn, Zn, Co, Ni, Fe and Ca are present in an amount between 13 to 16,5%, Mg is present in an amount between 6.0 to 7.0% (solid form).

12. A method of preparing a formulation for agronomical application comprising mixing a chelate A as defined in claim 1 and a chelate B as defined in claim 4.

13. The method of claim 3, 8 or 12, wherein at least one of the Cu, Mn, Zn, Fe and Ca are present in an amount between 5 to 7%, Mg is present in an amount between 1.5 to 3.5%, Co and Ni are present in an amount between 3.5 to 5.5% (liquid form).

14. The method of claim 3, 8 or 12, wherein at least one of the Cu, Mn, Zn, Co, Ni, Fe and Ca are present in an amount between 13 to 16,5%, Mg is present in an amount between 6.0 to 7.0% (solid form).

## Patentansprüche

1. Chelat A mit der Struktur [M(Ligand 1) (Ligand 2)]2Y, wobei:
M ein Metall ist, ausgewählt aus der Gruppe bestehend aus Cu, Mn, Zn, Co, Ni, Mg, Ca und Fe;
Ligand 1 Glutaminsäure ist;
Ligand 2 Apfelsäure ist; und
Y ein basisches Kation ist, wobei die Base ausgewählt ist aus der Gruppe bestehend aus NaOH, KOH und/oder NH4OH,
wobei ein Bindemittelverhältnis von Glutaminsäure zu Apfelsäure zu Metall 1:1:1 beträgt.

2. Formulierung zur agronomischen Applikation, wobei die Formulierung Folgendes umfasst:
ein Chelat A mit der Struktur [M(Ligand 1) (Ligand 2)]2Y, wobei:
M ein Metall ist, ausgewählt aus der Gruppe bestehend aus Cu, Mn, Zn, Co, Ni, Mg, Ca und Fe;
Ligand 1 Glutaminsäure ist;
Ligand 2 Apfelsäure ist; und
Y ein basisches Kation ist, wobei die Base ausgewählt ist aus der Gruppe bestehend aus NaOH, KOH und/oder NH4OH; und
Wasser,
wobei das Bindemittelverhältnis von Glutaminsäure zu Apfelsäure zu Metall 1:1:1 beträgt.

3. Verfahren zum Applizieren einer agronomischen Formulierung, wobei das Verfahren Folgendes beinhaltet:
Herstellen einer Formulierung, die ein wasserlösliches Chelat A mit der Struktur [M(Ligand 1) (Ligand 2)]2Y umfasst, wobei:
M ein Metall ist, ausgewählt aus der Gruppe bestehend aus Cu, Mn, Zn, Co, Ni, Mg, Ca und Fe;
Ligand 1 Glutaminsäure ist;
Ligand 2 Apfelsäure ist; und
Y ein basisches Kation ist, wobei die Base ausgewählt ist aus der Gruppe bestehend aus NaOH, KOH und/oder NH4OH;
wobei das Bindemittelverhältnis von Glutaminsäure zu Apfelsäure zu Metall 1:1:1 beträgt; und
Applizieren der Formulierung durch mindestens eines von einer Blattapplikation, einer Saatgutapplikation und einer Bodenapplikation.

4. Chelat B mit der Struktur [M(Ligand 1) (Ligand 2)]2Y, wobei:
M ein Metall ist, ausgewählt aus der Gruppe bestehend aus Cu, Mn, Zn, Co, Ni, Mg, Ca und Fe;
Ligand 1 Glutaminsäure ist;
Ligand 2 Asparaginsäure ist; und
Y ein basisches Kation ist, wobei die Base ausgewählt ist aus der Gruppe bestehend aus NaOH und/oder KOH; und
wobei das Bindemittelverhältnis von Glutaminsäure zu Apfelsäure zu Metall 1:1:1 beträgt.

5. Chelat nach Anspruch 1 oder 4, wobei mindestens eines von Cu, Mn, Zn, Fe und Ca in einer Menge zwischen 5 und 7 % vorhanden ist, Mg in einer Menge zwischen 1,5 und 3,5 % vorhanden ist, Co und Ni in einer Menge zwischen 3,5 und 5,5 % (in flüssiger Form) vorhanden sind.

6. Chelat nach Anspruch 1 oder 4, wobei mindestens eines von Cu, Mn, Zn, Co, Ni, Fe und Ca in einer Menge zwischen 13 und 16,5 % und Mg in einer Menge zwischen 6,0 und 7,0 % (in fester Form) vorhanden ist.

7. Formulierung zur agronomischen Applikation, wobei die Formulierung Folgendes umfasst:
ein Chelat B mit der Struktur [M(Ligand 1) (Ligand 2)]2Y, wobei:
M ein Metall ist, ausgewählt aus der Gruppe bestehend aus Cu, Mn, Zn, Co, Ni, Mg, Ca und Fe;
Ligand 1 Glutaminsäure ist;
Ligand 2 Asparaginsäure ist; und
Y ein basisches Kation ist, wobei die Base ausgewählt ist aus der Gruppe bestehend aus NaOH, KOH und/oder NH4OH; und
Wasser;
wobei das Bindemittelverhältnis von Glutaminsäure zu Apfelsäure zu Metall 1:1:1 beträgt.

8. Verfahren zum Applizieren einer agronomischen Formulierung, wobei das Verfahren Folgendes beinhaltet:
Herstellen einer Formulierung, die ein wasserlösliches Chelat B mit der Struktur [M(Ligand 1) (Ligand 2)]2Y umfasst, wobei:
M ein Metall ist, ausgewählt aus der Gruppe bestehend aus Cu, Mn, Zn, Co, Ni, Mg, Ca und Fe;
Ligand 1 Glutaminsäure ist;
Ligand 2 Asparaginsäure ist; und
Y ein basisches Kation ist, wobei die Base ausgewählt ist aus der Gruppe bestehend aus NaOH, KOH und/oder NH4OH;
wobei das Bindemittelverhältnis von Glutaminsäure zu Apfelsäure zu Metall 1:1:1 beträgt; und
Applizieren der Formulierung durch mindestens eines von einer Blattapplikation, einer Saatgutapplikation und einer Bodenapplikation.

9. Formulierung zur agronomischen Applikation, wobei die Formulierung eine Mischung aus einem Chelat A gemäß Anspruch 1 und einem Chelat B gemäß Anspruch 4 umfasst.

10. Formulierung nach Anspruch 2, 7 oder 9, wobei mindestens eines von Cu, Mn, Zn, Fe und Ca in einer Menge zwischen 5 und 7 % vorhanden ist, Mg in einer Menge zwischen 1,5 und 3,5 % vorhanden ist, Co und Ni in einer Menge zwischen 3,5 und 5,5 % (in flüssiger Form) vorhanden sind.

11. Formulierung nach Anspruch 2, 7 oder 9, wobei mindestens eines von Cu, Mn, Zn, Co, Ni, Fe und Ca in einer Menge zwischen 13 und 16,5 % und Mg in einer Menge zwischen 6,0 und 7,0 % (in fester Form) vorhanden ist.

12. Verfahren zur Herstellung einer Formulierung zur agronomischen Applikation, das das Mischen eines Chelats A gemäß Anspruch 1 und eines Chelats B gemäß Anspruch 4 beinhaltet.

13. Verfahren nach Anspruch 3, 8 oder 12, wobei mindestens eines von Cu, Mn, Zn, Fe und Ca in einer Menge zwischen 5 und 7 % vorhanden ist, Mg in einer Menge zwischen 1,5 und 3,5 % vorhanden ist, Co und Ni in einer Menge zwischen 3,5 und 5,5 % (in flüssiger Form) vorhanden sind.

14. Verfahren nach Anspruch 3, 8 oder 12, wobei mindestens eines von Cu, Mn, Zn, Co, Ni, Fe und Ca in einer Menge zwischen 13 und 16,5 % und Mg in einer Menge zwischen 6,0 und 7,0 % (in fester Form) vorhanden ist.

## Revendications

1. Chélate A ayant une structure [M(Ligand 1)(Ligand 2)]2Y, dans lequel :
M est un métal sélectionné parmi le groupe composé de Cu, Mn, Zn, Co, Ni, Mg, Ca et Fe ;
le ligand 1 est de l'acide glutamique ;
le ligand 2 est de l'acide malique ; et
Y est un cation de base, dans lequel la base est sélectionnée parmi le groupe composé de NaOH, KOH et/ou de NH4OH,
dans lequel un rapport proportionnel de liants d'acide glutamique à acide malique à métal est de 1:1:1.

2. Formulation pour application agronomique, la formulation comprenant :
un chélate A ayant une structure [M(Ligand 1)(Ligand 2)]2Y, dans lequel :
M est un métal sélectionné parmi le groupe composé de Cu, Mn, Zn, Co, Ni, Mg, Ca et Fe ;
le ligand 1 est de l'acide glutamique ;
le ligand 2 est de l'acide malique ; et
Y est un cation de base, dans lequel la base est sélectionnée parmi le groupe composé de NaOH, KOH et/ou de NH4OH ; et
de l'eau,
dans lequel un rapport proportionnel de liants d'acide glutamique à acide malique à métal est de 1:1:1.

3. Procédé d'application d'une formulation agronomique, le procédé comprenant :
préparer une formulation comprenant un chélate A soluble dans l'eau ayant une structure [M(Ligand 1)(Ligand 2)]2Y, dans lequel :
M est un métal sélectionné parmi le groupe composé de Cu, Mn, Zn, Co, Ni, Mg, Ca et Fe ;
le ligand 1 est de l'acide glutamique ;
le ligand 2 est de l'acide malique ; et
Y est un cation de base, dans lequel la base est sélectionnée parmi le groupe composé de NaOH, KOH et/ou de NH4OH,
dans lequel un rapport proportionnel de liants d'acide glutamique à acide malique à métal est de 1:1:1 ; et
appliquer la formulation via au moins l'une d'entre une application foliaire, une application à des graines et une application au sol.

4. Chélate B ayant une structure [M(Ligand 1)(Ligand 2)]2Y, dans lequel :
M est un métal sélectionné parmi le groupe composé de Cu, Mn, Zn, Co, Ni, Mg, Ca et Fe ;
le ligand 1 est de l'acide glutamique ;
le ligand 2 est de l'acide aspartique ; et
Y est un cation de base, dans lequel la base est sélectionnée parmi le groupe composé de NaOH et/ou de NH4OH,
dans lequel un rapport proportionnel de liants d'acide glutamique à acide malique à métal est de 1:1:1.

5. Chélate selon la revendication 1 ou 4, dans lequel au moins l'un du Cu, Mn, Zn, Fe et Ca est présent en une quantité de 5 à 7 %, Mg est présent en une quantité d'entre 1,5 à 3,5 %, Co et Ni sont présents en une quantité d'entre 3,5 et 5,5 % (forme liquide).

6. Chélate selon la revendication 1 ou 4, dans lequel au moins l'un du Cu, Mn, Zn, Co, Ni, Fe et Ca est présent en une quantité d'entre 13 et 16,5 %, Mg est présent en une quantité d'entre 6,0 et 7,0 % (forme solide).

7. Formulation pour application agronomique, la formulation comprenant :
un chélate B ayant une structure [M(Ligand 1)(Ligand 2)]2Y, dans lequel :
M est un métal sélectionné parmi le groupe composé de Cu, Mn, Zn, Co, Ni, Mg, Ca et Fe ;
le ligand 1 est de l'acide glutamique ;
le ligand 2 est de l'acide aspartique ; et
Y est un cation de base, dans lequel la base est sélectionnée parmi le groupe composé de NaOH, KOH et/ou de NH4OH, et
de l'eau ;
dans lequel un rapport proportionnel de liants d'acide glutamique à acide malique à métal est de 1:1:1.

8. Procédé d'application d'une formulation agronomique, le procédé comprenant :
préparer une formulation comprenant un chélate B soluble dans l'eau ayant une structure [M(Ligand 1)(Ligand 2)]2Y, dans lequel :
M est un métal sélectionné parmi le groupe composé de Cu, Mn, Zn, Co, Ni, Mg, Ca et Fe ;
le ligand 1 est de l'acide glutamique ;
le ligand 2 est de l'acide aspartique ; et
Y est un cation de base, dans lequel la base est sélectionnée parmi le groupe composé de NaOH, KOH et/ou de NH4OH,
dans lequel un rapport proportionnel de liants d'acide glutamique à acide malique à métal est de 1:1:1 ; et
appliquer la formulation via au moins l'une d'entre une application foliaire, une application à des graines et une application au sol.

9. Formulation pour application agronomique, la formulation comprenant un mélange d'un chélate A selon la revendication 1 et d'un chélate B selon la revendication 4.

10. Formulation selon la revendication 2, 7 ou 9, dans laquelle au moins l'un du Cu, Mn, Zn, Fe et Ca est présent en une quantité de 5 à 7 %, Mg est présent en une quantité d'entre 1,5 à 3,5 %, Co et Ni sont présents en une quantité d'entre 3,5 et 5,5 % (forme liquide).

11. Formulation selon la revendication 2, 7 ou 9, dans laquelle au moins l'un du Cu, Mn, Zn, Co, Ni, Fe et Ca est présent en une quantité d'entre 13 et 16,5 %, Mg est présent en une quantité d'entre 6,0 et 7,0 % (forme solide).

12. Procédé de préparation d'une formulation pour application agronomique comprenant mélanger un chélate A selon la revendication 1 et un chélate B selon la revendication 4.

13. Procédé selon la revendication 3, 8 ou 12, dans lequel au moins l'un du Cu, Mn, Zn, Fe et Ca est présent en une quantité de 5 à 7 %, Mg est présent en une quantité d'entre 1,5 à 3,5 %, Co et Ni sont présents en une quantité d'entre 3,5 et 5,5 % (forme liquide).

14. Procédé selon la revendication 3, 8 ou 12, dans lequel au moins l'un du Cu, Mn, Zn, Co, Ni, Fe et Ca est présent en une quantité d'entre 13 et 16,5 %, Mg est présent en une quantité d'entre 6,0 et 7,0 % (forme solide).
